(19) Europäisches Patentamt
European Patent Office
Office européen des brevets

(11) Publication number: **0 141 221 A1**

# (12) EUROPEAN PATENT APPLICATION

(21) Application number: 84111185.9

(22) Date of filing: 19.09.84

(51) Int. Cl.⁴: C 07 F 9/65
A 61 K 31/675

(30) Priority: 26.09.83 JP 177710/83
20.07.84 JP 151782/84

(43) Date of publication of application:
15.05.85 Bulletin 85/20

(84) Designated Contracting States:
AT BE CH DE FR GB IT LI LU NL SE

(71) Applicant: NISSAN CHEMICAL INDUSTRIES LTD.
7-1, 3-chome Kanda-Nishiki-cho
Chiyoda-ku Tokyo(JP)

(72) Inventor: Seto, Kiyotomo Nissan Chemical Industries Ltd.
Chuo Kenkyusho 722-1, Tsuboi-cho
Funabashi-shi Chiba-ken(JP)

(72) Inventor: Tanaka, Sakuya Nissan Chemical Industries Ltd.
Seibutsu Kagaku Kenkyusho 1470, Oaza-Shiraoka
Shiraoka-machi Ninami-Saitamagun Saitama(JP)

(72) Inventor: Sakoda, Ryozo c/o Nissan Chemical Industries, Ltd.
Chuo Kenkyusho 722-1, Tsuboi-cho
Funabashi-shi Chiba-ken(JP)

(74) Representative: Wächtershäuser, Günter, Dr.
Tal 29
D-8000 München 2(DE)

## (54) 1,4-Dihydropyridine-5-phosphonic acid ester.

(57) A 1,4-dihydropyridine-5-phosphonic acid ester represented by the general formula:

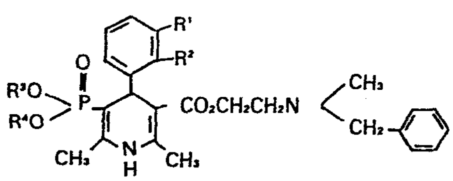

(I)

where $R^1$ is a hydrogen atom, a chlorine atom, a nitro group or a trifluoromethyl group, $R^2$ is a hydrogen atom, a chlorine atom or a trifluoromethyl group, and each of $R^3$ and $R^4$ is an alkyl group having 1 to 10 carbon atoms or a methoxyethyl group, or its pharmaceutically acceptable salt.

EP 0 141 221 A1

1,4-DIHYDROPYRIDINE-5-PHOSPHONIC ACID ESTER

The present invention relates to a novel 1,4-dihydropyridine-5-phosphonic acid ester, a process for the preparation thereof, and an antihypertensive or coronary or peripheral vasodilator composition containing the ester or its pharmaceutically acceptable salt.

Namely, the present invention provides a novel 1,4-dihydropyridine-5-phosphonic acid ester represented by the general formula:

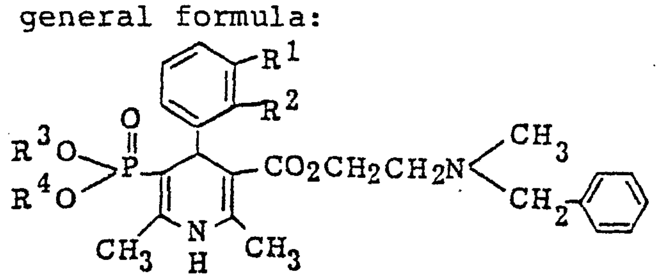

(I)

where $R^1$ is a hydrogen atom, a chlorine atom, a nitro group or a trifluoromethyl group, $R^2$ is a hydrogen atom, a chlorine atom or a trifluoromethyl group, and each of $R^3$ and $R^4$ is an alkyl group having 1 to 10 carbon atoms or a methoxyethyl group, or its pharmaceutically acceptable salt.

The compound of the formula I or its pharmaceutically acceptable salt according to the present invention is useful as an antihypertensive drug or as a coronary or peripheral vasodilator.

Some of the compounds of the formula I have optical isomers or diastereomers. The present invention covers such optical isomers and diastereomers.

Further, the present invention provides a process for producing the compound of the formula I, which comprises reacting a phosphonate derivative represented by the general formula:

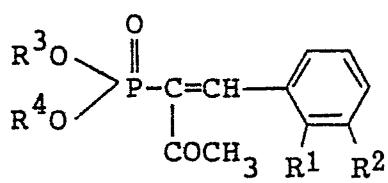

(II)

where $R^1$, $R^2$, $R^3$ and $R^4$ are as defined above, with a compound represented by the formula:

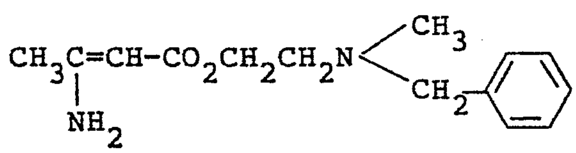

(III)

and optionally converting the resulting compound of the formula I to its pharmaceutically acceptable salt.

The starting compound of the formula II may be obtained by reacting a β-ketophosphonate derivative with an aldehyde in accordance with a conventional technique. Likewise, the starting compound of the formula III can readily be obtained by reacting the corresponding carbonyl compound with ammonia. The starting compound of the formula III may be formed in the reaction system by

mixing the corresponding carbonyl compound with ammonia and is not necessarily required to be isolated.

The reaction of the phosphonate derivative of the formula II with the compound of the formula III is usually conducted in an inert solvent. The inert solvent includes an alcohol solvent such as methanol, ethanol, propanol or isopropanol, an ether solvent such as 1,2-dimethoxyethane or THF, an aromatic hydrocarbon solvent such as benzene, toluene or xylene, a nitrile solvent such as acetonitrile or benzonitrile, an amido solvent such as DAM, DMF or N-methylpyrrolidone, a sulfoxide solvent such as DMSO or sulfolane, an ester solvent such as ethyl acetate or butyrolactone, or pyridine.

The reaction is usually conducted at a temperature of from room temperature to 200°C, preferably from 60 to 140°C, for from 1 to 100 hours, preferably from 5 to 20 hours.

1,4-Dihydropyridines are known to be useful for the medical treatment of coronary heart diseases, cerebral diseases, hypertension or arrhythmia, as they are capable of inhibiting the contraction of smooth muscle and cardiac muscle by calcium antagonistic effects (see A. Fleckenstein, Annu. Rev. Pharmacol. Toxicol., 17, 149-166 (1977)). However, the majority of 1,4-dihydropyridines known or being developed are substituted at the 3- and 5-positions by a carboxylic acid ester group. Further, these 1,4-dihydropyridines, such as Nicardipine, have a

side effect of tachycardia, and the duration of their antihypertensive effect is relatively short.

The present inventors have studied dihydropyridines. As a result, it has been unexpectedly found that the 1,4-dihydropyridine-5-phosphonate derivatives of the present invention are not only effective for inhibiting the contraction of cardiac muscle by the calcium antagonistic effects but also substantially free from the effect to increase the heart rate i.e. the side effect of tachycardia, which is observed with Nicardipine. Further, some of the compounds of the present invention have a longer antihypertensive effectiveness than Nicardipine.

1,4-Dihydropyridine-5-phosphonate derivatives are disclosed in a few prior art references. However, none of them suggests or indicates the specific compounds of the present invention.

Namely, A.I. Razumov et al. synthesized a 1,4-dihydropyridine-4-alkyl-5-phosphonate derivative (Zh. Obshch. Khim., 47, 1190-1191 (1977) and ibid., 51, 547-552 (1981)). Further, Von K. Issleib et al. synthesized 1,4-dihydropyridine-4-aryl-5-phosphonates (more specifically, diethyl 2,6-dimethyl-4-phenyl-3-ethoxycarbonyl-1,4-dihydropyridine-5-phosphonate and diethyl 2,6-dimethyl-4-(4-methoxyphenyl)-3-ethoxy-carbonyl-1,4-dihydropyridine-5-phosphonate) (J. Prakt. Chem., Vol. 318, 207-220 (1976)). None of these references indicates the pharmacological activities.

Furthermore, U.K. Patent Application GB 2105989A discloses a wide range of 1,4-dihydropyridine-5-phosphonate derivatives by a general formula, and teaches that the compounds represented by the general formula have cardiac activities. However, the specific compounds of the present invention and their specific activities are not disclosed or suggested by the U.K. Patent Application.

As mentioned above, the compounds of the present invention are not only capable of inhibiting the contraction of smooth muscle and cardiac muscle by the calcium antagonistic effects but also antihypertensively effective when administered orally. Thus, they are useful for the medical treatment of the coronary heart diseases, cerebral diseases or hypertension of mammals.

Thus, the present invention provides an antihypertensive agent and coronary or peripheral vasodilator composition comprising an effective amount of the compound of the formula I or its pharmaceutically acceptable salt, and a pharmaceutically acceptable diluent or carrier. Such a composition may also be formulated into a veterinary composition by combining the compound of the present invention with a veterinarily acceptable diluent or carrier.

Such compositions may be used in the form suitable for oral administration, e.g. tablets or capsules, in the form suitable for transdermal administration, e.g.

ointments or plasters, in the form suitable for inhalation, e.g. aerosols or solutions suitable for spraying, in the form suitable for injection administration, e.g. a sterilized aqueous solution, or in the form of a suppository suitable for use in anus, vagina or rectum.

The composition of the present invention usually contains the compound of the formula I in an amount of from about 0.1 to about 99.5% by weight, preferably from about 0.5 to about 95% by weight, based on the total weight of the composition.

The compound of the present invention or the composition of the present invention may be used in combination with other pharmaceutically or veterinarily active compounds. Further, the composition of the present invention may contain a plurality of the compounds of the formula I.

The daily dose of the compound of the formula I may be varied depending upon the type and the condition of the desease to be cured and the type of the patient (the age, sex, sensitivity, etc.). In the case of the intravenous administration, the daily dose is usually from 0.0001 to 10 mg, preferably from 0.0005 to 1 mg, of the active ingredient per 1 kg of the body weight. Likewise, in the case of the oral or transdermal administration, the daily dose is usually from 0.001 to 100 mg of the active ingredient per 1 kg of the body

weight. Further, the daily dose in the case of the administration in the form of a suppository to e.g. a vagina or rectum, is usually from 0.001 to 200 mg, preferably from 0.005 to 100 mg, of the active ingredient per 1 kg of the body weight. The content of the active ingredient in an aerosol, is usually from 0.1 to 10% by weight, preferably from 0.1 to 2% by weight. Such a daily dose may be divided for administration twice or more times per day.

The above-mentioned composition containing the compound of the formula I may be prepared by a conventional method, and a conventional excipient may be incorporated therein.

Referring to the formula I, it is preferred that $R^3$ is an alkyl group having 2 to 8 carbon atoms and $R^4$ is an alkyl group having 1 to 3 carbon atoms or a methoxy ethyl group. In another preferred embodiment, $R^1$ or $R^2$ is a hydrogen atom. Likewise, in a further preferred embodiment, $R^3$ is an ethyl group and $R^4$ is an methyl or ethyl group. In a still further preferred embodiment, $R^3$ is a n-butyl group and $R^4$ is a methyl group. In another preferred embodiment, $R^3$ is a n-hexyl group, and $R^4$ is a methyl or β-methoxyethyl group. In still another preferred embodiment, each of $R^3$ and $R^4$ is an i-propyl group.

Now, the present invention will be described in further detail with reference to Test Examples, Working Examples and various formulations. However, it should be understood that the present invention is by no means restricted by these specific Examples. In the following Examples, Me represents a methyl group, Et represents an ethyl group, Pr represents a propyl group, Bu represents a butyl group, Hex represents a hexyl group, and Oct represents an octyl group.

For ready reference, the structures of the compounds obtained by the Examples are shown in the following Table.

| Example No. | $R^1$ | $R^2$ | $R^3$ | $R^4$ |
|---|---|---|---|---|
| 1 | $NO_2$ | H | Et | Et |
| 2 | $CF_3$ | H | Et | Et |
| 3 | H | Cl | Et | Et |
| 4 | Cl | H | Et | Et |
| 5 | $NO_2$ | H | Et | Me |
| 6 | H | $CF_3$ | Et | Me |
| 7 | Cl | H | Et | Me |
| 8 | H | Cl | Et | Me |
| 9 | H | $CF_3$ | n-Bu | Me |
| 10 | Cl | H | n-Bu | Me |
| 11 | H | Cl | n-Bu | Me |
| 12 | H | $CF_3$ | n-Hex | Me |
| 13 | Cl | H | n-Hex | Me |
| 14 | H | Cl | n-Hex | Me |
| 15 | Cl | H | n-Oct | Me |
| 16 | H | $CF_3$ | i-Pr | i-Pr |
| 17 | $CF_3$ | H | i-Pr | i-Pr |
| 18 | H | Cl | i-Pr | i-Pr |
| 19 | Cl | H | i-Pr | i-Pr |
| 20 | $NO_2$ | H | n-Hex | $MeOCH_2CH_2$ |
| 21 | H | $CF_3$ | n-Hex | $MeOCH_2CH_2$ |
| 22 | Cl | H | n-Hex | $MeOCH_2CH_2$ |
| 23 | H | Cl | n-Hex | $MeOCH_2CH_2$ |
| 24 | H | $CF_3$ | Et | Et |

N-methyl-β-amino-ethoxycarbonyl)-1,4-... methyl-4-(3-nitrophenyl)-pyridine-5-phosphonate

1.64 g of diethyl α-acetyl-3-nitrostyrylphosphonate and 1.24 g of β-(N-benzyl-N-methylamino)ethyl 2-aminocrotonate were dissolved in 30 ml of toluene, and the solution was refluxed for 10 hours. During this period, the formed water was removed by azeotropic distillation. The solvent was distilled off under reduced pressure, and the residue was purified by silica gel column chromatography, whereby the above-mentioned compound was obtained.

Yield: 33%, yellow oily substance

MS, m/e (intensity ratio)

91(14) 166(58) 167(100) 540(7) 557 (2)

NMR ($CDCl_3$):

δ8.20-7.07(10H, m), 6.12(1H, broad s), 4.86(1H, d, J=8Hz), 4.38-3.52(6H, m), 3.50(2H, s), 2.55(2H, t, J=8Hz), 2.44(3H, s), 2.39(3H, s), 2.20(3H, s), 1.36-0.92(6H, m)

The above compound was dissolved in EtOH, and after an addition of 30% HCl-EtOH, the solvent was distilled off under reduced pressure, whereby the hydrochloride was obtained.

Compounds of Examples 2 to 4 were obtained in the same manner as in Example 1 by using the corresponding diethyl α-acetyl-substituted styryl phosphonates, respectively.

EXAMPLE 2:

Synthesis of diethyl 3-(N-benzyl-N-methyl-β-aminoethoxycarbonyl)-1,4-dihydro-2,6-dimethyl-4-(3-trifluoromethylphenyl)-pyridine-5-phosphonate

Yield: 41%, yellow oily substance

MS, m/e (intensity ratio):

134(75) 147(100) 148(40) 288(10) 580(8)

NMR ($CDCl_3$):

δ7.62-7.13(10H, m), 6.35(1H, broad s), 4.77(1H, d, J=11Hz), 4.25-3.53(6H, m), 3.48(2H, s), 2.62(2H, t, J=7Hz), 2.30(3H, s), 2.25(3H, s), 1.35-0.80(6H, m)

EXAMPLE 3:

Synthesis of 3-(N-benzyl-N-methyl-β-aminoethoxycarbonyl-1,4-dihydro-2,6-dimethyl-4-(2-chlorophenyl)-pyridine-5-phosphonate

Yield: 60%, yellow oily substance

MS, m/e (intensity ratio):

134(57) 147(100) 148(73) 288(38) 398(14) 546(10)

NMR ($CDCl_3$):

δ7.56-6.80(10H, m), 5.13(1H, d, J=10Hz), 4.30-3.07(6H, m), 3.42(2H, s), 2.61(2H, t, J=7Hz), 2.23(6H, s), 2.12(3H, s), 1.23(3H, t,J=8Hz), 0.87(3H, t, J=8Hz)

EXAMPLE 4:

Synthesis of diethyl 3-(N-benzyl-N-methyl-β-aminoethoxycarbonyl)-1,4-dihydro-2,6-dimethyl-4-(3-chlorophenyl)-pyridine-5-phosphonate

Yield: 36%, yellow oily substance

MS, m/e (intensity ratio):

134(50) 147(100) 148(55) 288(12) 546(14)

NMR ($CDCl_3$):

δ7.36-6.98(10H, m), 6.05(1H, broad s), 4.68(1H, d, J=12Hz), 4.27-3.55(6H, m), 3.49(2H, s), 2.63(2H, t, J=7Hz), 2.29(3H, s), 2.26(3H, s), 2.20(3H, s), 1.37-0.88(6H, m)

EXAMPLE 5:

Synthesis of O-ethyl-O'-methyl 3-(N-benzyl-N-methyl-β-aminoethoxycarbonyl)-1,4-dihydro-2,6-dimethyl-4-(3-nitrophenyl)-pyridine-5-phosphonate

0.52 g of O-ethyl-O'-methyl α-acetyl-3-nitrostyrylphosphonate and 0.26 g of β-(N-benzyl-N-methylamino)ethyl 2-aminocrotonate were dissolved in 5 ml of toluene, and the solution was refluxed for 4 hours. The solvent was distilled off under reduced pressure, and the residue was subjected to silica gel column chromatography. Then, the fraction containing the desired substance was concentrated, whereby the above-identified compound was obtained.

Yield: 54%, yellow oily substance

0141221

MS, m/e (intensity ratio):

91(20) 134(93) 147(100) 148(24) 543(2, $M^+$)

NMR ($CDCl_3$):

δ8.28-7.89(9H, m), 4.87(1H, d, J=7Hz), 4.22(2H, 5, J=5Hz), 3.55(3H, d, J=8Hz), 3.51(2H, s), 3.35(3H, t, J=8Hz), 4.30-3.38(2H, m), 2.66(2H, t, J=5Hz), 2.46-2.10(9H, m), 1.43-0.87(6H, m)

EXAMPLES 6 to 19:

O-$R^3$-O'-$R^4$ 3-[(N-methyl-N-benzyl)-β-amino-ethoxy-carbonyl]-1,4-dihydro-2,6-dimethyl-4-substituted phenylpyridine-5-phosphonates were synthesized in the same manner as in Example 5 except that various O-$R^3$-O'-$R^4$ α-acetyl-styryl-phosphonates [$(R^3O)(R^4O)P(O)C(C(O)CH_3)=CH$— 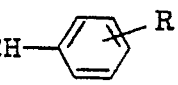 ] where $R^3$ and $R^4$ are as identified in Table 1 were used instead of O-ethyl-O'-methyl α-acetyl-3-nitrostyryl phosphonate, and the reflux time was as shown in Table 1.

The results of the syntheses are shown in Table 1.

Table 1

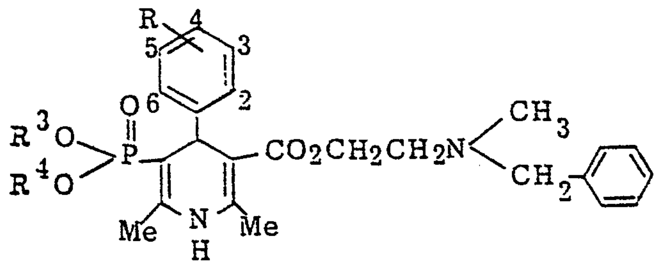

| Example Nos. | $R^3$ | $R^4$ | R | Reflux time (hr) | Yield (%) | Characteristics |
|---|---|---|---|---|---|---|
| 6 | Et | Me | 2-$CF_3$ | 7 | 21 | Yellow oily substance |
| 7 | Et | Me | 3-Cl | 16 | 40 | " |
| 8 | Et | Me | 2-Cl | 16 | 48 | " |
| 9 | n-Bu | Me | 2-$CF_3$ | 17 | 23 | " |
| 10 | n-Bu | Me | 3-Cl | 17 | 56 | " |
| 11 | n-Bu | Me | 2-Cl | 17 | 34 | " |
| 12 | n-Hex | Me | 2-$CF_3$ | 16 | 26 | " |
| 13 | n-Hex | Me | 3-Cl | 16 | 54 | " |
| 14 | n-Hex | Me | 2-Cl | 16 | 38 | " |
| 15 | n-Oct | Me | 3-Cl | 32 | 28 | " |
| 16 | i-Pr | i-Pr | 2-$CF_3$ | 49 | 56 | " |
| 17 | i-Pr | i-Pr | 3-$CF_3$ | 27 | 60 | " |
| 18 | i-Pr | i-Pr | 2-Cl | 46 | 27 | " |
| 19 | i-Pr | i-Pr | 3-Cl | 35 | 42 | " |

The MS and NMR spectra of the respective compounds are as follows.

EXAMPLE 6:

MS, m/e (intensity ratio):

91(21) 134(65) 147(100) 148(42) 274(11) 566(2, $M^+$)

NMR ($CDCl_3$):

δ 7.80-7.00(9H, m), 5.32(1H, d, J=8Hz), 4.33-3.92(5H, m), 3.50(2H, s), 3.01(3H, d, J=8Hz), 2.63(2H, t, J=4Hz), 2.99-2.07(9H, m), 1.51-0.68(6H, m)

EXAMPLE 7:

MS m/e (intensity ratio):

91(14) 134(48) 147(100) 148(41) 532(2, $M^+$)

NMR ($CDCl_3$):

δ 7.50-7.00(9H, m), 4.82(1H, d, J=7Hz), 4.20(2H, t, J=4Hz), 3.53(3H, d, J=7Hz), 3.53(2H, s), 3.49(3H, d, J=7Hz), 2.64(2H, t, J=4Hz), 2.46-2.20(9H, m), 1.22(3H, t, J=5Hz), 1.04(3H, t, J=4Hz)

EXAMPLE 8:

MS, m/e (intensity ratio):

91(16) 134(72) 147(100) 148(70) 274(14) 288(16) 546(3, $M^+$)

NMR ($CDCl_3$):

δ 7.58-6.80(9H, m), 5.16(1H, d, J=7Hz), 4.18(2H, t, J=5Hz), 3.47(2H, s), 3.48(3H, d, J=8Hz), 3.10(3H, d, J=8Hz), 2.63(2H, t, J=4Hz), 2.46-2.11(9H, m), 1.36(3H, t, J=5Hz), 0.92(3H, t, J=5Hz)

EXAMPLE 9:

MS, m/e (intensity ratio):

91(28) 134(73) 147(100) 148(61) 302(11)

NMR ($CDCl_3$):

δ7.80-7.03(9H, m), 5.30(1H, d, J=7Hz), 4.49-3.50(4H, m), 3.52(3H, d, J=8Hz), 3.51(2H, s), 3.03(3H, d, J=8Hz), 2.54(2H, t, J=5Hz), 2.98-2.16(9H, m), 1.81-0.69(7H, m)

EXAMPLE 10:

MS, m/e (intensity ratio):

91(12) 134(23) 147(100) 148(54) 560(6, $M^+$)

NMR ($CDCl_3$):

δ7.57-6.95(9H, m), 4.74(1H, d, J=7Hz), 4.20(2H, t, J=4Hz), 3.54(3H, d, J=7Hz), 3.52(2H, s), 3.30(3H, d, J=7Hz), 2.68(2H, t, J=4Hz), 2.50-2.13(9H, m), 1.76-0.64(7H, m)

EXAMPLE 11:

MS, m/e (intensity ratio):

134(50) 147(100) 148(55) 302(23) 412(15) 560(11, $M^+$)

NMR ($CDCl_3$):

δ7.60-6.96(9H, m), 6.65(1H, broad s), 5.67(1H, d, J=6Hz), 4.20(2H, t, J=4Hz), 3.61(3H, d, J=7Hz), 3.49(2H, s), 3.12(3H, d, J=7Hz), 2.67(2H, t, J=4Hz), 2.46-2.10(9H, m), 1.80-0.68(7H, m)

EXAMPLE 12:

MS, m/e (intensity ratio):

134(25) 147(100) 148(46) 330(15) 477(12) 622(3, $M^+$)

NMR ($CDCl_3$):

δ 7.82-6.95(9H, m), 5.29(1H, d, J=7Hz), 4.40-3.39(4H, m), 3.19(3H, d, J=8Hz), 3.50(2H, s), 3.02(3H, d, J=8Hz), 2.53(2H, t, J=5Hz), 2.48-2.14(9H, m), 1.34-0.62(11H, m)

EXAMPLE 13:

MS, m/e (intensity ratio):

134(52) 147(100) 148(43) 588(3, $M^+$)

NMR ($CDCl_3$):

δ 7.57-6.78(4H, m), 7.59(5H, s), 4.65(1H, d, J=10Hz), 4.17(2H, t, J=6Hz), 3.99-3.17(7H, m), 2.66(2H, t, J=6Hz), 2.45-2.15(9H, m), 1.70-0.66(11H, m)

EXAMPLE 14:

MS, m/e (intensity ratio):

91(17) 134(38) 147(100) 148(57) 330(18) 440(12) 588(8, $M^+$)

NMR ($CDCl_3$):

δ 7.65-6.85(9H, m), 5.16(1H, d, J=7Hz), 4.17(2H, t, J=5Hz), 3.10(3H, d, J=8Hz), 3.49(2H, s), 3.11(3H, d, J=8Hz), 2.65(3H, t, J=5Hz), 2.46-2.15(9H, m), 1.53-0.66(11H, m)

EXAMPLE 15:

MS, m/e (intensity ratio):

134(45) 147(100) 358(9) 468(5) 616(5, $M^+$)

NMR ($CDCl_3$):

δ7.43-6.86(4H, m), 7.23(5H, s), 4.71(1H, d, J=10Hz), 4.17(2H, t, J=6Hz), 3.96-3.15(7H, m), 2.65(2H, t, J=6Hz), 2.45-2.05(9H, m), 1.73-0.65(15H, m)

EXAMPLE 16:

MS, m/e (intensity ratio):

134(24) 147(100) 148(74) 316(28) 463(29) 608(6, $M^+$)

NMR ($CDCl_3$):

δ 7.72-6.90(9H, m), 5.31(1H, d, J=10Hz), 4.64-3.83(4H, m), 3.42(2H, broad s), 2.62(2H, t, J=4Hz), 2.43-2.15(9H, m), 1.40-0.85(12H, m)

EXAMPLE 17:

MS, m/e (intensity ratio):

134(57) 147(100) 148(33) 316(4) 608(4, $M^+$)

NMR ($CDCl_3$):

δ 7.76-7.70(9H, m), 4.85(1H, d, J=10Hz), 4.61-3.91(4H, m), 3.47(2H, s), 2.63(2H, t, J=6Hz), 2.42-2.03(9H, m), 1.43-0.83(12H, m)

EXAMPLE 18:

MS, m/e (intensity ratio):

134(80) 147(100) 148(93) 316(37) 463(12) 574(8, $M^+$)

NMR ($CDCl_3$):

δ 7.57-7.75(4H, m), 7.16(5H, s), 5.16(1H, d, J=10Hz), 4.57-3.88(4H, m), 3.44(2H, broad s), 2.62(2H, t, J=6Hz), 2.40-2.07(9H, m), 1.47-0.72(12H, m)

EXAMPLE 19:

MS, m/e (intensity ratio):

134(51) 147(100) 316(8) 463(3)

NMR ($CDCl_3$):

δ 7.3(5H, s), 7.3-6.9(4H, m), 6.65(1H, broad s), 4.75(1H, d, J=10Hz), 4.6-4.2(2H, m), 4.15(2H, t, J=6Hz), 3.45(2H, s), 2.6(2H, t, J=6Hz), 2.4-2.15(9H, m), 1.4-0.85(12H, m)

EXAMPLE 20:

<u>Synthesis of O-n-hexyl-O'-(β-methoxyethyl) 3-(N-benzyl-N-methyl-β-aminoethoxycarbonyl)-1,4-dihydro-2,6-dimethyl-4-(3-nitrophenyl)-pyridine-5-phosphonate</u>

0.88 g of O-n-hexyl-O'-methoxyethyl- α-acetyl-(3-nitrostyryl)-phosphonate and 0.25 g of β-(N-benzyl-N-methylamino)ethyl 2-aminocrotonate were dissolved in 20 ml of toluene, and the solution was refluxed for 20 hours. The solvent was distilled off under reduced pressure, and the residue was subjected to silica gel column chromatography by using 10% ethanol-ethyl acetate as the developer. The fraction containing the desired substance was distilled under reduced pressure to remove the solvent, whereby 0.77 g of the above-identified compound was obtained.

In a similar manner, compounds of Examples 22 to 24 were obtained. The yields and the characteristics are shown in Table 2. The spectrum data are shown in Table 2-2.

Table 2

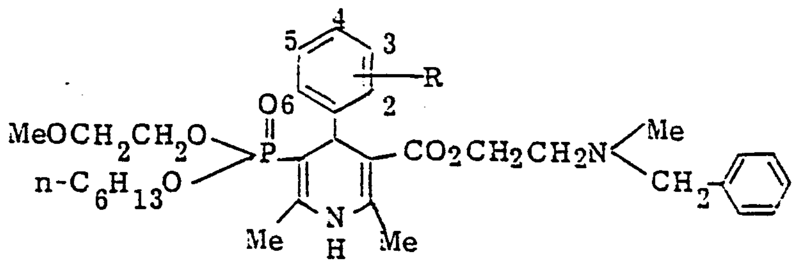

| Example Nos. | R | Yield (%) | Characteristics |
|---|---|---|---|
| 20 | 3-$NO_2$ | 56 | Yellow oily substance |
| 21 | 2-$CF_3$ | 37 | " |
| 22 | 3-Cl | 72 | " |
| 23 | 2-Cl | 59 | " |

Table 2-2: Spectrum data of the compounds of Table 2

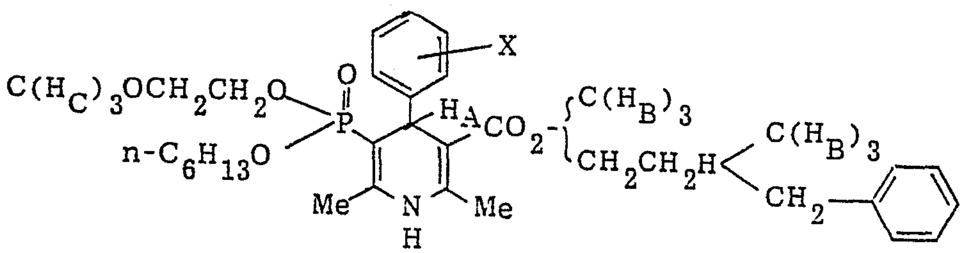

| Example Nos. | NMR spectrum ($CDCl_3$) | | | MS spectrum |
|---|---|---|---|---|
| | $\delta H_A$ | $\delta H_B$ | $\delta H_C$ | m/e (intensity ratio) |
| 20 | 4.90 | 2.20 | 3.32, 3.26 | 134(53) 147(100) 634(6, $M^+$) |
| 21 | 5.35 | 2.20 | 3.34 3.17 | 134(68) 147(100) 374(17) 666(4, $M^+$) |
| 22 | 4.76 | 2.22 | 3.33, 3.27 | 134(46) 147(100) 374(14) 632(13, $M^+$) |
| 23 | 5.14 | 2.13 | 3.28, 3.17 | 147(100) 374(55) 484(27) 632(27, $M^+$) |

EXAMPLE 24:

Synthesis of diethyl 3-(N-benzyl-N-methyl-β-aminoethoxycarbonyl)-1,4-dihydro-2,6-dimethyl-4-(2-trifluoromethylphenyl)-pyridine-5-phosphonate

1.75 g of diethyl α-acetyl-o-trifluoromethylstyryl phosphonate and 1.24 g of β-(N-benzyl-N-methylamino)-ethyl 2-aminocrotonate were dissolved in 30 ml of toluene, and the solution was refluxed for 10 hours. During this period, the formed water was removed by azeotropic distillation. The solvent was distilled off under reduced pressure, and the residue was purified by silica gel column chromatography, whereby the above-identified compound was obtained.

Yield: 39%, yellow oily substance

MS, m/e (intensity ratio):

147(100) 288(23) 435(15) 580(2)

NMR ($CDCl_3$):

δ 8.0-6.8(10H, m), 5.2(1H, d, J=12Hz), 4.4-3.3(6H, m), 3.4(2H, s), 2.6(2H, t, J=8Hz), 2.4-2.1(9H, m), 1.5-0.6(6H, m)

Test 1: Calcium antagonistic effects

10 mm in situ length of taenia caecum of guinea pig was suspended at a tension of 1 g in a 20 ml organ bath filled with a physiological salt solution (NaCl: 135mM, KCl: 5mM etc.).

This solution was bubbled with a gas mixture of 95% $O_2$-5% $CO_2$ and kept at 37°C. Then, the preparation was

depolarized by a $K^+$ rich solution (NaCl: 40mM, KCl: 100 mM). After 10-20 minutes equilibration period, 10mM of $CaCl_2$ was added to the bathing solution. The contraction was produced and then the test compound was applied cumulatively. The relaxation produced was expressed as percentage of the maximum relaxation produced by $10^{-4}$ M papaverine, and the concentration of the compound producing 50% relaxation, i.e. $ID_{50}$(M), was calculated. The values of $pID_{50}$ ($pID_{50}=-\log[ID_{50}]$) are summarized in Table 3.

Test 2: Effects on blood pressure and heart rate

(a) Measurement of antihypertensive effects (i.v.)

Male spontaneously hypertensive rats (SHR) were anesthetized intraperitoneally with a mixture of urethane (600 mg/kg) and α-chloralose (60 mg/kg).

After dilution with physiological saline containing 3% Tween 80, the compound was injected into the right femoral vein. The arterial blood pressure of SHR was measured from the left carotid artery, and the dose induced 30% decrease of the blood pressure, i.e. $ED_{30}$(mg/kg) are summarized in Table 3.

(b) Effects on blood pressure and heart rate (p.o.)

After oral administration of Nicardipine (30 mg/kg) or the test compound (60 mg/kg) dissolved in a $H_2O$ - PEG 400 solvent mixture ($H_2O$:PEG400(W/W)=1:3) to male SHR, the systolic blood pressure and heart rate were measured by a tail cuff method. Prior to the measurement, rats

were warmed at 50°C for 5 minutes. The results are summarized in Table 3.

Test 3: Acute toxicity test

ddY mice (♂, 4 weeks old) were divided into groups of five mice and the test compound dissolved in purified water was administered orally (5% solution) or intraperitoneally (1% solution) to the male ddY mice.

After seven days, $LD_{50}$ values were calculated from the dead rats recorded in the individual dosage groups by the method of Litchfield-Wilcoxon. The results are shown in Table 3.

Table 3 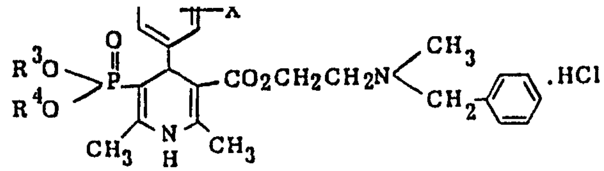

| Example No. | X | $R^3$ | $R^4$ | Calcium antagonistic effects | Effects on blood pressure and heart rate | Changes in blood pressure and heart rate | | | | Actute toxicity | |
|---|---|---|---|---|---|---|---|---|---|---|---|
| | | | | | | | | | | $LD_{50}$ | |
| | | | | $pID_{50}$ | $ED_{30}$ | 2 hr | 4 hr | 6 hr | 8 hr | i.p. | p.o. |
| Nicardi-pine (b) | | | | 9.38 | 0.026 | +++<br>↑↑ | +++<br>↑↑ | +++<br>↑↑ | +<br>↑↑ | 166 | 561 |
| 1 | $3-NO_2$ | Et | Et | 7.74 | 0.27 | +++<br>– | +++<br>– | +++<br>– | +++<br>– | | |
| 2 | $3-CF_3$ | Et | Et | 7.49 | 0.13 | +++<br>– | +++<br>– | +++<br>– | +++<br>– | 262 | 648 |
| 3 | 2-Cl | Et | Et | 7.15 | 0.15 | ++<br>– | ++<br>– | ++<br>– | –<br>– | | |
| 4 | 3-Cl | Et | Et | 7.58 | 0.20 | +<br>– | ++<br>– | +++<br>– | ++<br>– | 138 | 464 |
| 5 | $3-NO_2$ | Me | Et | 7.38 | 0.27 | ++<br>↑ | ++<br>↑ | +++<br>↑ | +<br>– | | |
| 6 | $2-CF_3$ | Me | Et | 8.36 | 0.099 | +++<br>– | +++<br>– | +++<br>– | +++<br>– | | |
| 7 | 3-Cl | Me | Et | 7.38 | 0.18 | +<br>– | ++<br>– | ++<br>– | +<br>– | | |
| 8 | 2-Cl | Me | Et | 7.26 | 0.23 | +<br>– | +<br>– | ++<br>– | +<br>↑ | | |
| 9 | $2-CF_3$ | Me | n-Bu | 8.19 | 0.22 | ++<br>– | +++<br>– | +++<br>– | +++<br>– | | |
| 10 | 3-Cl | Me | n-Bu | 8.08 | 0.23 | +<br>– | ++<br>– | ++<br>– | +<br>– | | |
| 11 | 2-Cl | Me | n-Bu | 7.54 | 0.19 | +<br>– | ++<br>↑ | +++<br>– | +<br>– | | |
| 12 | $2-CF_3$ | Me | n-Hex | 7.55 | 0.22 | +<br>– | ++<br>↑ | ++<br>↑ | –<br>– | | |

Table 3 (cont'd)

| Example No. | X | $R^3$ | $R^4$ | Calcium antagonistic effects | Effects on blood pressure and heart rate | | | | | Actute toxicity | |
|---|---|---|---|---|---|---|---|---|---|---|---|
| | | | | | | Changes in blood pressure and heart rate | | | | $LD_{50}$ | |
| | | | | $pID_{50}$ | $ED_{30}$ | 2 hr | 4 hr | 6 hr | 8 hr | i.p. | p.o. |
| 13 | 3-Cl | Me | n-Hex | 5.57 | 0.85 | + ↑ | ++ ↑ | +++ ↑ | +++ ↑ | | |
| 14 | 2-Cl | Me | n-Hex | 7.42 | 0.21 | + − | ++ ↑ | +++ ↑ | + ↑ | | |
| 15 | 3-Cl | Me | n-Octyl | 6.83 | 0.21 | + ↑ | +++ ↑ | ++ ↑ | + ↑ | | |
| 16 | $2-CF_3$ | i-Pr | i-Pr | 7.54 | 0.14 | +++ − | +++ − | +++ − | +++ − | 113 | |
| 17 | $3-CF_3$ | i-Pr | i-Pr | 7.44 | 0.17 | ++ − | ++ − | ++ − | − − | | |
| 18 | 2-Cl | i-Pr | i-Pr | 6.97 | 0.19 | + − | ++ ↓ | + − | − − | | |
| 19 | 3-Cl | i-Pr | i-Pr | 7.42 | 0.085 | + − | ++ ↓ | ++ − | + − | | |
| 20 | $3-NO_2$ | n-Hex | $MeOCH_2CH_2$ | 8.19 | 0.16 | +++ ↑ | +++ ↑ | +++ ↑ | +++ ↑ | | |
| 21 | $2-CF_3$ | n-Hex | $MeOCH_2CH_2$ | 8.04 | 0.21 | +++ ↑ | +++ ↑ | +++ ↑ | +++ ↑ | | |
| 22 | 3-Cl | n-Hex | $MeOCH_2CH_2$ | 7.37 | 0.20 | + − | +++ − | +++ − | + − | | |
| 23 | 2-Cl | n-Hex | $MeOCH_2CH_2$ | 7.90 | 0.23 | ++ − | +++ ↑ | +++ ↑ | +++ − | | |
| 24 | $2-CF_3$ | Et | Et | 7.53 | 0.29 | +++ − | +++ − | +++ − | +++ − | | |

Now, examples will be given for various formulations containing the compound of the formula I.

Tablets

Composition (1,000 tablets)

| | |
|---|---|
| Hydrochloride of the compound of the Example 2 | 5.0 (g) |
| Lactose | 190.0 |
| Corn starch | 75.0 |
| Crystal cellulose powder | 25.0 |
| Methyl cellulose | 3.0 |
| Magnesium stearate | 2.0 |
| | 300.0 |

The above ingredients in the respective amounts were introduced into a twin shell mixer and uniformly mixed. This powder mixture was tableted by a direct compression method to obtain tablets having a weight of 300 mg per tablet.

Capsules

Composition (1,000 capsules)

| | |
|---|---|
| Hydrochloride of the Compound of the Example 2 | 5 (g) |
| Corn starch | 145 |
| Crystal cellulose powder | 145 |
| Magnesium stearate | 5 |
| | 300 |

The above ingredients in the respective amounts were introduced into a twin shell mixer and uniformly mixed. This powder mixture was packed in hard gelatin capsules in an amount of 300 mg per capsule.

Powder

Composition:

| | |
|---|---|
| Hydrochloride of the compound of the Example 2 | 1.0 (g) |
| Lactose | 88.0 |
| Crystal cellulose powder | 10.0 |
| Methyl cellulose | 1.0 |
| | 100.0 |

The above ingredients in the respective amounts were introduced into a twin shell mixer and uniformly mixed to obtain a powder.

Syrups

Composition (2% syrups):

| | |
|---|---|
| Hydrochloride of the compound of the Example 2 | 2.0 (g) |
| Sugar | 30.0 |
| Glycerin | 5.0 |
| Flavoring agent | 0.1 |
| 96% ethanol | 10.0 |
| Methyl p-hydroxybenzoate | 0.03 |
| Purified water | to make 100.0 g |

0141221

The sugar and the hydrochloride of the compound of Example 2 were dissolved in 60 g of warm water, and after cooling the solution, a solution of the flavoring agent in glycerin and ethanol was added. Then, water was added to this mixture to bring the total amount to 100.0 g.

CLAIMS:

1. A 1,4-dihydropyridine-5-phosphonic acid ester represented by the general formula:

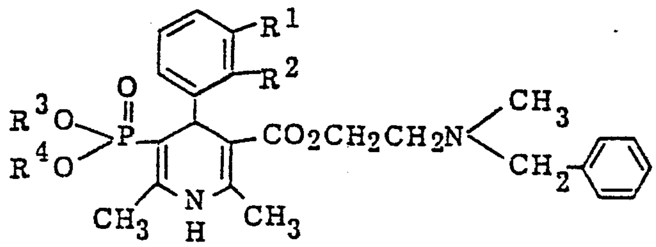

(I)

where $R^1$ is a hydrogen atom, a chlorine atom, a nitro group or a trifluoromethyl group, $R^2$ is a hydrogen atom, a chlorine atom or a trifluoromethyl group, and each of $R^3$ and $R^4$ is an alkyl group having 1 to 10 carbon atoms or a methoxyethyl group, or its pharmaceutically acceptable salt.

2. The 1,4-dihydropyridine-5-phosphonic acid ester or its pharmaceutically acceptable salt according to Claim 1, wherein $R^3$ is an alkyl group having 2 to 8 carbon atoms, and $R^4$ is an alkyl group having 1 to 3 carbon atoms or a methoxyethyl group.

3. The 1,4-dihydropyridine-5-phosphonic acid ester or its pharmaceutically acceptable salt according to Claim 1, wherein $R^1$ or $R^2$ is a hydrogen atom.

4. The 1,4-dihydropyridine-5-phosphonic acid ester or its pharmaceutically acceptable salt according to Claim 1, wherein $R^3$ is an ethyl group, and $R^4$ is an methyl or ethyl group.

5. The 1,4-dihydropyridine-5-phosphonic acid ester or its pharmaceutically acceptable salt according to Claim 1, wherein $R^3$ is a n-butyl group, and $R^4$ is a methyl group.

6. The 1,4-dihydropyridine-5-phosphonic acid ester or its pharmaceutically acceptable salt according to Claim 1, wherein $R^3$ is a n-hexyl group, and $R^4$ is a methyl or β-methoxyethyl group.

7. The 1,4-dihydropyridine-5-phosphonic acid ester or its pharmaceutically acceptable salt according to Claim 1, wherein each of $R^3$ and $R^4$ is an i-propyl group.

8. A process for producing a compound represented by the general formula:

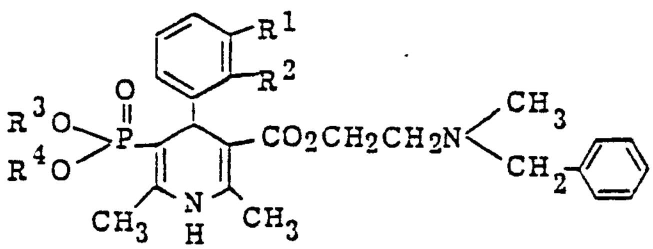

(I)

where $R^1$ is a hydrogen atom, a chlorine atom, a nitro group or a trifluoromethyl group, $R^2$ is a hydrogen atom, a chlorine atom or a trifluormethyl group, and each of $R^3$ and $R^4$ is an alkyl group having 1 to 10 carbon atoms or a methoxyethyl group, or its pharmaceutically acceptable salt, which comprises reacting a phosphonate derivative represented by the general formula:

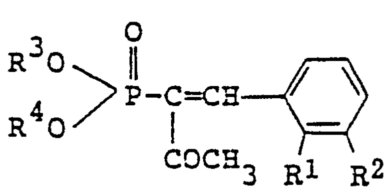

(II)

where $R^1$, $R^2$, $R^3$ and $R^4$ are as defined above, with a compound represented by the formula:

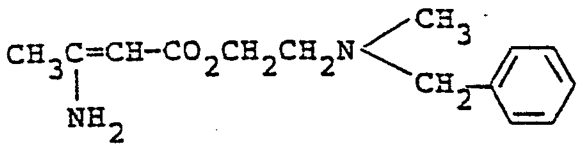

(III)

and optionally converting the resulting compound of the formula I to its pharmaceutically acceptable salt.

9. An antihypertensive composition or coronary or peripheral vasodilator composition comprising an effective amount of a 1,4-dihydropyridine-5-phosphonic acid ester of the formula I as defined in Claim 1 or its pharmaceutically acceptable salt, and a pharmaceutically acceptable diluent or carrier.

10. The 1,4-dihydropyridine-5-phosphonic acid ester of the formula I as defined in Claim 1 or its pharmaceutcially acceptable salt for use as an active antihypertensive agent or coronary or peripheral vasodilator agent.

0141221

European Patent Office

# EUROPEAN SEARCH REPORT

Application number

EP 84 11 1185

| Category | Citation of document with indication, where appropriate, of relevant passages | Relevant to claim | CLASSIFICATION OF THE APPLICATION (Int. Cl.4) |
|---|---|---|---|
| D,A | EP-A-0 071 819 (BAYER AG) * claims * | 1,9,10 | C 07 F 9/65<br>A 61 K 31/675 |
| | --- | | |
| A | US-A-3 485 847 (F. BOSSERT) * column 2, lines 24-32; claims * | 1,9,10 | |
| | --- | | |
| E | EP-A-0 121 117 (NIPPON SHINYAKU CO. LTD.) * examples 1,5,11,20,22,27,31,39,42,46,48,50,52,54,56,58,60,62,65,67,71; claims * | 1-10 | |
| | ----- | | |
| | | | TECHNICAL FIELDS SEARCHED (Int. Cl.4) |
| | | | C 07 F 9/00 |

The present search report has been drawn up for all claims

| Place of search | Date of completion of the search | Examiner |
|---|---|---|
| THE HAGUE | 19-12-1984 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82